# EUROPEAN PATENT APPLICATION

(11) **EP 2 143 738 A1**
(43) Date of publication of application: **13.01.2010**
(21) Application number: 08159936.7
(22) Date of filing: 08.07.2008
(51) Int. Cl.: C08F 14/18, C07D 317/10

(54) **Method for manufacturing fluoropolymers**

(71) Applicant: Solvay Solexis S.p.A., 20121 Milano (IT)
(72) Inventor: Marchionni, Giuseppe, 20133, MILANO (IT); Tortelli, Vito, 20137, MILANO (IT); Wlassics, Ivan, 12075, Garessio (CN) (IT); Kapeliouchko, Valeri, 15100, Alessandria (IT)
(74) Representative: Benvenuti, Federica

(57) **Abstract**

The invention pertains to a method for making a fluoropolymer comprising an aqueous emulsion polymerization of one or more fluorinated monomers wherein said aqueous emulsion polymerization is carried out in the presence of at least one cyclic fluorocompound of the following formula (I): 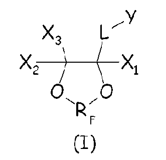 wherein X₁, X₂, X₃, equal or different from each other are independently selected among H, F, and C₁₋₆ (per)fluoroalkyl groups, optionally comprising one or more catenary or non-catenary oxygen atoms; L represents a bond or a divalent group; R_{F} is a divalent fluorinated C₁₋₃ bridging group; Y is a hydrophilic function selected among anionic functionalities, cationic functionalities and non-ionic functionalities.

## Description

### Technical Field

The present invention pertains to a method of making fluoropolymer dispersions, to fluoropolymer dispersions therefrom and to cyclic fluorosurfactants useful in said method.

### Background Art

Fluoropolymers, i.e. polymers having a fluorinated backbone, have been long known and have been used in a variety of applications because of several desirable properties such as heat resistance, chemical resistance, weatherability, UV-stability etc.

A frequently used method for producing fluoropolymers involves aqueous emulsion polymerization of one or more fluorinated monomers generally involving the use of fluorinated surfactants. Frequently used fluorinated surfactants include perfluorooctanoic acids and salts thereof, in particular ammonium perfluorooctanoic acid.

Recently, perfluoroalkanoic acids having 8 or more carbon atoms have raised environmental concerns. For instance, perfluoroalkanoic acids have been found to show bioaccumulation. Accordingly, efforts are now devoted to phasing out from such compounds and methods have been developed to manufacture fluoropolymer products using alternative surfactants having a more favourable toxicological profile.

Several approaches have been recently pursued to this aim, typically involving fluorosurfactants comprising a perfluoroalkyl chain interrupted by one or more catenary oxygen atoms, said chain having an ionic carboxylate group at one of its ends.

Examples of these compounds which are endowed with improved bioaccumulation profile over perfluoro alkanoic acids having 8 or more carbon atoms can be found notably in US 2007276103 (3M INNOVATIVE PROPERTIES CO ) 29.11.2007, US 2007015864 (3M INNOVATIVE PROPERTIES CO ) 18.01.2007 , US 2007015865 (3M INNOVATIVE PROPERTIES CO) 18.01.2007 , US 2007015866 (3M INNOVATIVE PROPERTIES CO ) 18.01.2007.

It would thus be desirable to find alternative fluorinated surfactants that can be used in the emulsion polymerization of fluorinated monomers which desirably show lower bioaccumulation/biopersistence than perfluoro alkanoic acids having 8 or more carbon atoms.

It would further be desirable that the surfactant properties of said alternative fluorinated surfactants be such that polymerization can be carried out in a convenient and cost effective way, using equipment commonly used in the aqueous emulsion polymerization of fluorinated monomers with traditional surfactants.

### Disclosure of Invention

It has been found that cyclic fluorocompounds of the following formula (I): 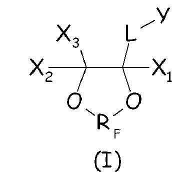 as detailed below, are effective in the aqueous emulsion polymerization, even when used without the addition of other surfactants such as perfluoroalkanoic acids and salts thereof.

Moreover, the Applicant has surprisingly found that above mentioned cyclic fluorocompounds (I) have significantly improved biopersistence behaviour over perfluoroalkanoic acids derivatives, so that their toxicological profile is much improved.

Finally, these cyclic fluorocompounds (I) have a higher volatility over perfluoroalkanoic acids derivatives, so that their residues in final parts obtained from fluoropolymer dispersions containing the same can be significantly reduced.

Thus, in one aspect, the invention relates to a method for making a fluoropolymer comprising an aqueous emulsion polymerization of one or more fluorinated monomers wherein said aqueous emulsion polymerization is carried out in the presence of at least one cyclic fluorocompound of the following formula (I): 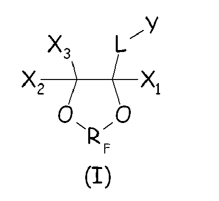 wherein X₁, X₂, X₃, equal or different from each other are independently selected among H, F, and C₁₋₆ (per)fluoroalkyl groups, optionally comprising one or more catenary or non-catenary oxygen atoms; L represents a bond or a divalent group; R_{F} is a divalent fluorinated C₁₋₃ bridging group; Y is a hydrophilic function selected among anionic functionalities, cationic functionalities and non-ionic functionalities.

The hydrophilic function Y can be notably selected among non-ionic functions of formulae -(OR_{H})ₙ-OH, wherein R_{H} is a divalent hydrocarbon group, and n is an integer of 1 to 15.

As an alternative, the hydrophilic function Y can be notably selected among cationic functions of formulae: 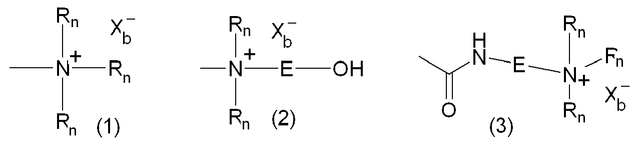 wherein Rₙ, equal or different at each occurrence, represents a hydrogen atom or a C₁₋₆ hydrocarbon group (preferably an alkyl group), E is a C₁₋₃ divalent hydrocarbon group and X_{b}⁻ is an anion selected among OH-, Cl-, Br-, I⁻.

Nevertheless, the hydrophilic function Y is preferably selected among anionic functions, in particular among those of formulae: 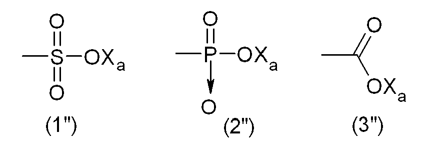 wherein Xₐ is H, a monovalent metal (preferably an alkaline metal) or an ammonium group of formula -N(R'ₙ)₄, wherein R'ₙ, equal or different at each occurrence, represents a hydrogen atom or a C₁₋₆ hydrocarbon group (preferably an alkyl group).

Most preferably, hydrophilic function Y is a carboxylate of formula (3"), as above detailed.

According to a first embodiment of the invention, the cyclic fluorocompound complies with formula (II) here below: 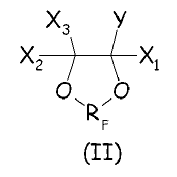 wherein X₁, X₂, X₃, Y and R_{F} have the same meaning as above defined.

More preferably, the cyclic fluorocompound complies with formula (III) here below: 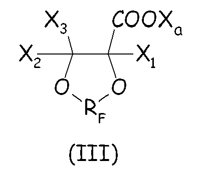 wherein R_{F}, X₁, X₂, X₃, and Xₐ have the same meaning as above defined.

According to a first variant of this preferred embodiment, the cyclic fluorocompound complies with formula (IV): 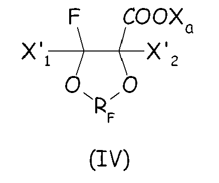 wherein X'₁ and X'₂ , equal or different from each other, are independently a fluorine atom, a -R'_{f} group or-OR'_{f} group, wherein R'_{f} is a C₁-₃ perfluoroalkyl group, and R_{F} and Xₐ have the same meanings as above defined.

Compounds of formula (IV) can be notably obtained by reaction of perfluoroallylfluorosulfate derivatives of formula: 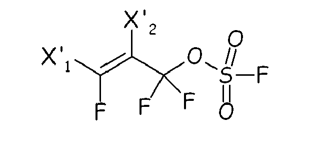 with a bis-hypofluorite of formula: 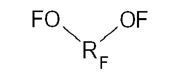 so as to obtain corresponding adduct of formula: 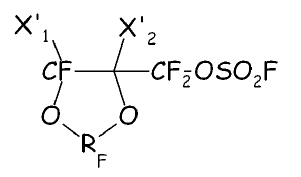 which yields by hydrolysis the target compound (IV).

Hydrolysis of above mentioned adduct is preferably accomplished by alkaline hydrolysis with an aqueous inorganic base, e.g. with aqueous KOH, optionally followed by treatment with an aqueous acidic solution (e.g. HCl_{aq}) for obtaining carboxylic acids and/or further neutralisation for introducing required counter-cation onto the carboxylic group.

In an alternative method for preparing cyclic fluorocompounds of formula (IV) here above, a cyclic fluoroolefin is reacted with carbonyl fluoride in the presence of fluorides, as sketched in scheme herein below: 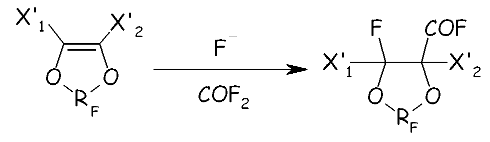 wherein X'₁, X'₂, R_{F} have the meaning as above defined.

So obtained carbonyl fluoride derivative can be easily hydrolized to yield the target compound (IV).

In a further alternative method, cyclic fluorocompound of formula (IV) can be prepared by adding to a cyclic fluoroolefin methanol for obtaining a cyclic fluorinated methanol derivative, as sketched in the scheme herein below: 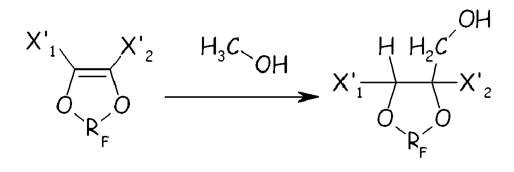 wherein X'₁ , X'₂ and R_{F} have the meaning as above defined and R'_{H} is H or a C₁₋₆ hydrocarbon group. Cyclic alcohol derivative can be further transformed in compound (IV) via following steps:
(i) esterification of the cyclic alcohol with a fluorinated acyl fluoride yielding the corresponding ester: 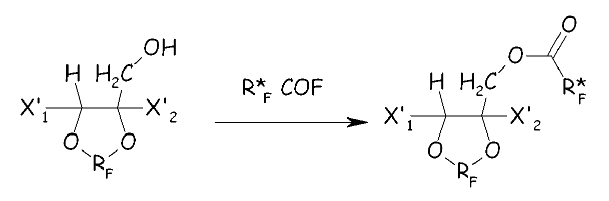
(ii) complete fluorination of all C-H bonds in C-F bonds of this latter to yield corresponding perfluorinated ester compound: 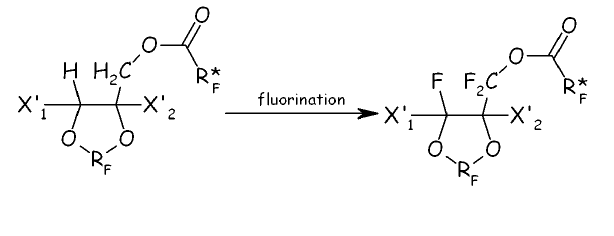
(iii) decomposition of the perfluoroester to yield the corresponding perfluoroacyl compound: 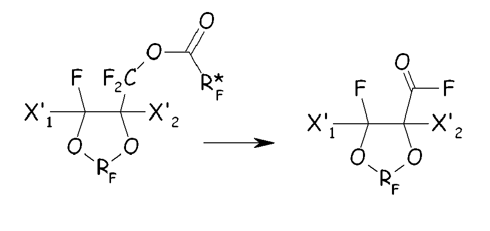
(iv) hydrolysis and treatment with a base for yielding the corresponding carboxylate derivative (IV):

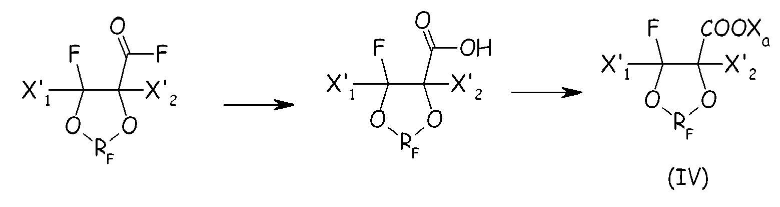

Any other process enabling complete fluorination of the C-H bonds, but preserving alcohol/carboxylic functionality under protected form can be also suitable for transforming above mentioned cyclic fluorinated methanol derivative in compound (IV).

The cyclic fluorocompound (IV) of the first variant of this preferred embodiment more preferably complies with formula (V): 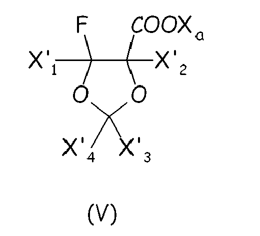 wherein X'₁, X'₂, X'₃, X'₄, equal or different each other are independently a fluorine atom, -R'_{f} or -OR'_{f}, wherein R'_{f} is a C₁₋₃ perfluoroalkyl group.

Non limitative examples of cyclic fluorocompounds of formula (V) are notably:

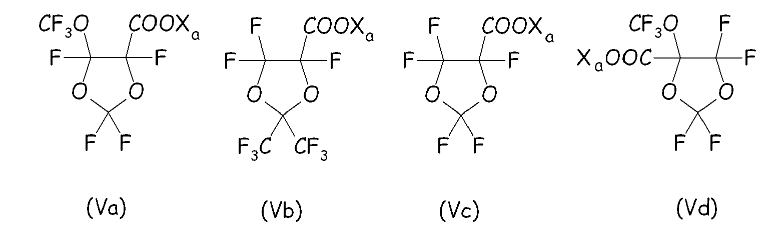

According to a second variant of this preferred embodiment, the cyclic fluorocompound complies with formula (VI) here below: 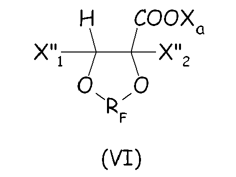 wherein X"₁ and X"₂, equal or different from each other, are independently a fluorine atom, a -R'_{f} group or -OR'_{f} group, wherein R'_{f} is a C₁-₃ perfluoroalkyl group, and R_{F} and Xₐ have the same meanings as above defined.

Cyclic fluorocompound of formula (VI) can be prepared by adding to a cyclic fluoroolefin a hydrocarbon primary alcohol for obtaining a cyclic fluorinated alcohol derivative, as sketched in the scheme herein below: 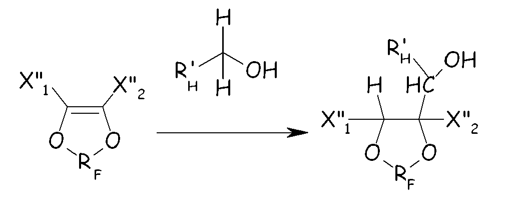 wherein X"₁ , X"₂ and R_{F} have the meaning as above defined and R'_{H} is H or a C₁₋₆ hydrocarbon group.

Suitable hydrocarbon alcohols include aliphatic alcohols such as lower alkanols having 1 to 4 carbon atoms. Specific examples include methanol, ethanol, propanol and butanol, methanol being particularly preferred.

The reaction of the fluorinated olefin with the alcohol may be carried out as described in CHAMBERS, R. D.Fluorine in Organic Chemistry. Oxford (UK): Blackwell Publishing, 2004. ISBN 0849317908. p.199 and ss..

The resulting cyclic fluorinated alcohol derivative can be chemically oxidized with an oxidizing agent to the corresponding carboxylic acid derivative (optionally followed by suitable hydrolysis/neutralisation steps), as depicted here below: 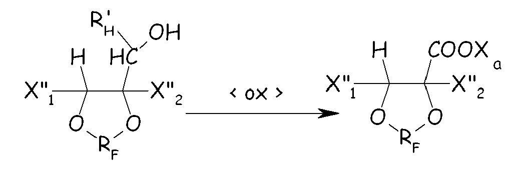 wherein X"₁, X"₂, R'_{H}, R_{F} and Xₐ have the same meanings as above defined.

Examples of oxidizing agents include for example potassium permanganate, chromium (VI) oxide, RuO₄ or OsO₄ optionally in the presence of NaOCl, nitric acid/iron catalyst, dinitrogen tetroxide. Typically the oxidation is carried out in acidic or basic conditions, preferably basic conditions, at a temperature between 10° and 100°C. In addition to chemical oxidation, electrochemical oxidation may be used as well.

The cyclic fluorocompound (VI) of the second variant of this preferred embodiment more preferably complies with formula (VII): 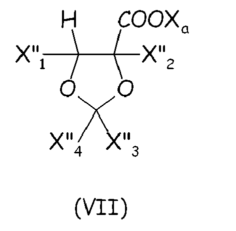 wherein X"₁, X"₂, X"₃, X"₄, equal or different each other are independently a fluorine atom, -R'_{f} or -OR'_{f}, wherein R'_{f} is a C₁₋₃ perfluoroalkyl group.

Non limitative examples of cyclic fluorocompounds of formula (VII) are notably:

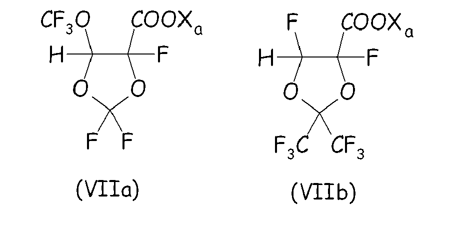

According to a second embodiment of the invention, the cyclic fluorocompound complies with formula (VIII) here below: 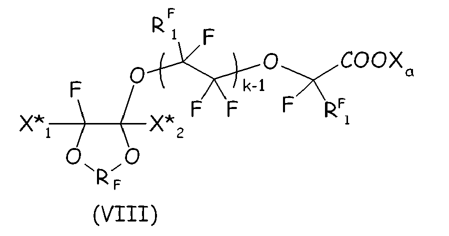 wherein R^{F} and Xₐ have the same meanings as above detailed; X*₁, X*₂ equal or different each other are independently a fluorine atom, -R'_{f} or -OR'_{f}, wherein R'_{f} is a C₁₋₃ perfluoroalkyl group; R^{F}₁ is F or CF₃, k is an integer from 1 to 3.

Compounds of formula (VIII) can be notably manufactured by reaction of an unsaturated fluorodioxole with a hydrogenated glycol derivative, as sketched herein below, so as to obtain a mono-addition compound of formula (X): 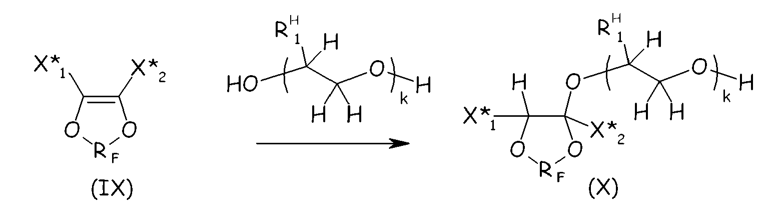 wherein X*₁, X*₂, R_{F}, k have the same meaning as above defined; R^{H}₁ is H or -CH₃.

Basic catalysis is generally adopted for favouring this reaction. Addition of the hydrogenated glycol derivative is generally carried out using 1 eq of said unsaturated dioxole (IX) per equivalent of base in said glycol, so as to maximize yield towards target mono-addition compound (X). As the hydroxyl functionality is generally unstable under fluorination conditions, the free hydroxyl group of the addition product (X) is generally protected before full fluorination: 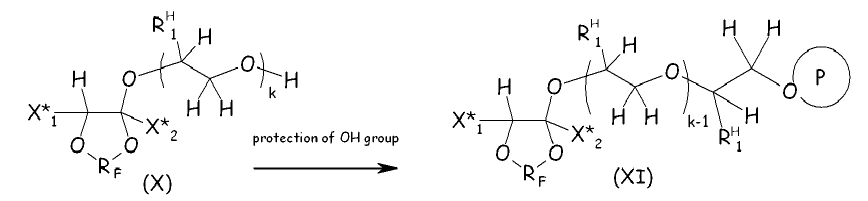 wherein X*₁, X*₂, R_{F}, R^{H}₁, k have the meaning above defined; and round circle P denotes a protecting group.
The choice of the protecting agent is not particularly limited, provided that this group is stable under fluorination conditions. Generally, an esterification with a (per)fluorinated acyl fluoride will be the preferred route.

The protected addition product (XI) is then fluorinated according to standard procedures, typically using elemental fluorine, to yield corresponding perfluorocompound (XII): 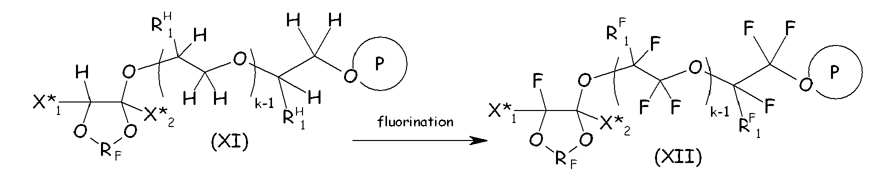 wherein X*₁, X*₂, R_{F}, R^{H}₁, R^{F}₁, k and round circle P have same meaning as above detailed.

Said perfluorocompound derivative (XII) is then submitted to appropriate reaction conditions for decomposing/hydrolyzing protecting group of the hydroxyl function, so as to yield corresponding acyl fluoride which is then converted by hydrolysis/neutralization in target compound (VIII): 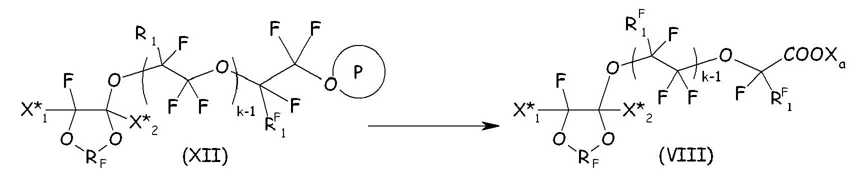 wherein X*₁, X*₂, R_{F}, R^{F}₁, Xₐ, k and round circle P have same meaning as above detailed.

This synthetic pathway can be notably applied with success for converting unsaturated perfluorodioxole of formulae: 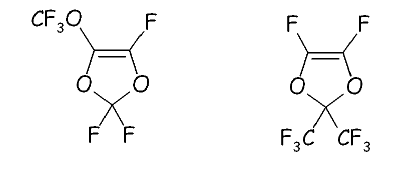 in corresponding cyclic fluorocompounds (VIII) by reaction with ethylene glycol, reaction with a fluoroacyl compound (e.g. (CF₃)₂-CF-COF) to yield corresponding ester, fluorination, decomposition of the ester and final hydrolysis/neutralization, as sketched in following scheme : 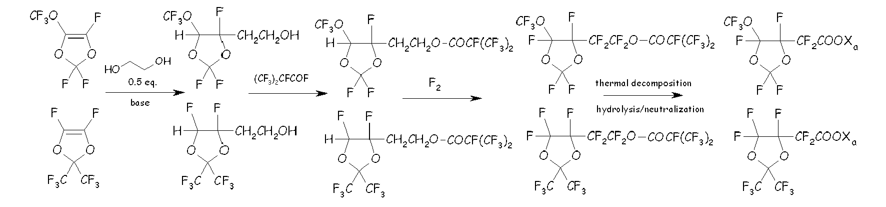 Perfluoroester can be notably broken to yield the corresponding acyl fluoride by thermal decomposition in the presence of a nucleophile or an electrophyle, typically in the presence of a metal fluoride of formula MeF_{y}, with Me being a metal having y valence, y being 1 or 2, in particular in the presence of NaF, CaF₂, AgF, CsF, KF, preferably KF.

In the process of the invention, one or more cyclic fluorocompound of formula (I) are used in the aqueous emulsion polymerization of one or more fluorinated monomers, in particular gaseous fluorinated monomers.

By gaseous fluorinated monomers is meant monomers that are present as a gas under the polymerization conditions. In a particular embodiment, the polymerization of the fluorinated monomers is started in the presence of the cyclic fluorocompound of formula (I), i.e. the polymerization is initiated in the presence of the same. The amount of cyclic fluorocompound of formula (I) used may vary depending on desired properties such as amount of solids, particle size etc.... Generally the amount of cyclic fluorocompound of formula (I) will be between 0.001 % by weight based on the weight of water in the polymerization and 5% by weight. A practical range is between 0.05% by weight and 1 % by weight.

While the polymerization is generally initiated in the presence of the cyclic fluorocompound of formula (I), it is not excluded to add further cyclic fluorocompound of formula (I) during the polymerization, although such will generally not be necessary.

Nevertheless, it may be desirable to add certain monomer to the polymerization in the form of an aqueous emulsion. For example, fluorinated monomers and in particular perfluorinated co-monomers that are liquid under the polymerization conditions may be advantageously added in the form of an aqueous emulsion. Such emulsion of such co-monomers is preferably prepared using cyclic fluorocompound of formula (I) as an emulsifier.

The aqueous emulsion polymerization may be carried out at a temperature between 10 to 150°C, preferably 20°C to 110°C and the pressure is typically between 2 and 50 bar, in particular 5 to 25 bar.

The reaction temperature may be varied during the polymerization e.g. for influencing the molecular weight distribution, i.e., to obtain a broad molecular weight distribution or to obtain a bimodal or multimodal molecular weight distribution.

The pH of the polymerization media may be in the range of pH 2-11, preferably 3-10, most preferably 4-10.

The aqueous emulsion polymerization is typically initiated by an initiator including any of the initiators known for initiating a free radical polymerization of fluorinated monomers. Suitable initiators include peroxides and azo compounds and redox based initiators. Specific examples of peroxide initiators include, hydrogen peroxide, sodium or barium peroxide, diacylperoxides such as diacetylperoxide, disuccinyl peroxide, dipropionylperoxide, dibutyrylperoxide, dibenzoylperoxide, benzoylacetylperoxide, diglutaric acid peroxide and dilaurylperoxide, and further per-acids and salts thereof such as e.g. ammonium, sodium or potassium salts. Examples of per-acids include peracetic acid. Esters of the peracid can be used as well and examples thereof include tert.-butylperoxyacetate and tert.-butylperoxypivalate. Examples of inorganic initiators include for example ammonium-alkali- or earth alkali salts of persulfates, permanganic or manganic acid or manganic acids. A persulfate initiator, e.g. ammonium persulfate (APS), can be used on its own or may be used in combination with a reducing agent. Suitable reducing agents include bisulfites such as for example ammonium bisulfite or sodium metabisulfite, thiosulfates such as for example ammonium, potassium or sodium thiosulfate, hydrazines, azodicarboxylates and azodicarboxyldiamide (ADA). Further reducing agents that may be used include sodium formaldehyde sulfoxylate (Rongalit ) or fluoroalkyl sulfinates as disclosed in U.S. Pat. No. 5,285,002. The reducing agent typically reduces the half-life time of the persulfate initiator. Additionally, a metal salt catalyst such as for example copper, iron or silver salts may be added. The amount of initiator may be between 0.01 % by weight (based on the fluoropolymer solids to be produced) and 1% by weight. In one embodiment, the amount of initiator is between 0.05 and 0.5% by weight. In another embodiment, the amount may be between 0.05 and 0.3% by weight.

The aqueous emulsion polymerization can be carried out in the presence of other materials, such as notably buffers and, if desired, complex-formers or chain-transfer agents.

Examples of chain transfer agents that can be used include dimethyl ether, methyl t-butyl ether, alkanes having 1 to 5 carbon atoms such as ethane, propane and n-pentane, halogenated hydrocarbons such as CCl₄, CHCl₃ and CH₂Cl₂ and hydrofluorocarbon compounds such as CH₂F-CF₃ (R134a). Additionally esters like ethylacetate, malonic esters can be effective as chain transfer agent in the process of the invention.

Examples of fluorinated monomers that may be polymerized using the cyclic fluorocompound according to formula (I) as an emulsifier in the process of the invention include partially or fully fluorinated gaseous monomers including fluorinated olefins such as tetrafluoroethylene (TFE), chlorotrifluoroethylene (CTFE), hexafluoropropylene (HFP), vinyl fluoride (VF), vinylidene fluoride (VDF), partially or fully fluorinated allyl ethers and partially or fully fluorinated vinyl ethers.

The polymerization may further involve non-fluorinated monomers such as ethylene and propylene.

Further examples of fluorinated monomer that may be used in the aqueous emulsion polymerization according to the invention include those corresponding to the formula: CF₂=CF-O-R_{f} wherein R_{f} represents a perfluorinated aliphatic group that may contain one or more oxygen atoms.

Still further, the polymerization may involve comonomers that have a functional group such as for example a group capable of participating in a peroxide cure reaction. Such functional groups include halogens such as Br or I as well as nitrile groups.

The aqueous emulsion polymerization may be used to produce a variety of fluoropolymers including perfluoropolymers, which have a fully fluorinated backbone, as well as partially fluorinated fluoropolymers. Also the aqueous emulsion polymerization may result in melt-processable fluoropolymers as well as those that are not melt-processable such as for example polytetrafluoroethylene and so-called modified polytetrafluoroethylene. The polymerization process can further yield fluoropolymers that can be cured to make fluoroelastomers as well as fluorothermoplasts. Fluorothermoplasts are generally fluoropolymers that have a distinct and well noticeable melting point, typically in the range of 60 to 320°C or between 100 and 320°C. They thus have a substantial crystalline phase. Fluoropolymers that are used for making fluoroelastomers typically are amorphous and/or have a neglectable amount of crystallinity such that no or hardly any melting point is discernable for these fluoropolymers.

The aqueous emulsion polymerization process of the invention results in a dispersion of the fluoropolymer in water comprising the cyclic fluorocompound of formula (I). Generally the amount of solids of the fluoropolymer in the dispersion directly resulting from the polymerization will vary between 3 % by weight and about 40% by weight depending on the polymerization conditions. A typical range is between 5 and 30% by weight, for example between 10 and 25% by weight.

The particle size (volume average diameter) of the fluoropolymer is typically between 40 nm and 400 nm with a typical particle size being between 60 nm and about 350 nm. The total amount of cyclic fluorocompound formula (I) in the resulting dispersion is typically between 0.001 and 5% by weight based on the amount of fluoropolymer solids in the dispersion. A typical amount may be from 0.01 to 2% by weight or from 0.02 to 1% by weight.

The fluoropolymer may be isolated from the dispersion by coagulation if a polymer in solid form is desired. Also, depending on the requirements of the application in which the fluoropolymer is to be used, the fluoropolymer may be post-fluorinated so as to convert any thermally unstable end groups into stable CF₃- end groups.

For coating applications, an aqueous dispersion of the fluoropolymer is desired and hence the fluoropolymer will not need to be separated or coagulated from the dispersion. To obtain a fluoropolymer dispersion suitable for use in coating applications such as for example in the impregnation of fabrics or in the coating of metal substrates to make for example cookware, it will generally be desired to add further stabilizing surfactants and/or to further increase the fluoropolymer solids. For example, non-ionic stabilizing surfactants may be added to the fluoropolymer dispersion. Typically these will be added thereto in an amount of 1 to 12 % by weight based on fluoropolymer solids. Examples of non-ionic surfactants that may be added include R¹-O-[CH₂CH₂O]ₙ-[R²O]ₘ -R³ (NS) wherein R¹ represents an aromatic or aliphatic hydrocarbon group having from 6 to 18 carbon atoms, R² represents an alkylene having 3 carbon atoms, R³ represents hydrogen or a C₁₋₃ alkyl group, n has a value of 0 to 40, m has a value of 0 to 40 and the sum of n+m being at least 2. It will be understood that in the above formula (NS), the units indexed by n and m may appear as blocks or they may be present in an alternating or random configuration. Examples of non-ionic surfactants according to formula (VI) above include alkylphenol oxy ethylates such as ethoxylated p-isooctylphenol commercially available under the brand name TRITON™ such as for example TRITON™ X 100 wherein the number of ethoxy units is about 10 or TRITON™ X 114 wherein the number of ethoxy units is about 7 to 8. Still further examples include those in which R¹ in the above formula (NS) represents an alkyl group of 4 to 20 carbon atoms, m is 0 and R³ is hydrogen. An example thereof includes isotridecanol ethoxylated with about 8 ethoxy groups and which is commercially available as GENAPOL^{®} X080 from Clariant GmbH. Non-ionic surfactants according to formula (NS) in which the hydrophilic part comprises a block-copolymer of ethoxy groups and propoxy groups may be used as well. Such non-ionic surfactants are commercially available from Clariant GmbH under the trade designation GENAPOL^{®} PF 40 and GENAPOL^{®} PF 80.

The amount of fluoropolymer solids in the dispersion may be upconcentrated as needed or desired to an amount between 30 and 70% by weight. Any of the known upconcentration techniques may be used including ultrafiltration and thermal upconcentration.

Still an object of the invention are fluoropolymer dispersions comprising at least one cyclic fluorocompound of formula (I), as above described.

Said fluoropolymer dispersions are typically obtained by the process of the invention.

Concentration of cyclic fluorocompound of formula (I) in the fluoropolymer dispersions of the invention can be reduced, if necessary, following traditional techniques. Mention can be made of ultrafiltration combined with percolate recycle, as described in US 4369266 (HOECHST AG ) 18.01.1983 , treatment with ion exchange resins in the presence of a non-ionic surfactant (as described in EP 1155055 A (DYNEON GMBH ) 21.11.2001 ), of an anionic surfactant (as exemplified in EP 1676868 A (SOLVAY SOLEXIS SPA) 05.07.2006 ) or of a polyelectrolyte (as taught in EP 1676867 A (SOLVAY SOLEXIS SPA) 05.07.2006 ).

Also, cyclic fluorocompounds as above detailed and processes for their manufacture are other objects of the present invention.

The invention will be now explained in more detail with reference to the following examples, whose purpose is merely illustrative and not intended to limit the scope of the invention.

### Preparative Example 1

**Synthesis of compound Vila (with Xₐ = NH₄)**

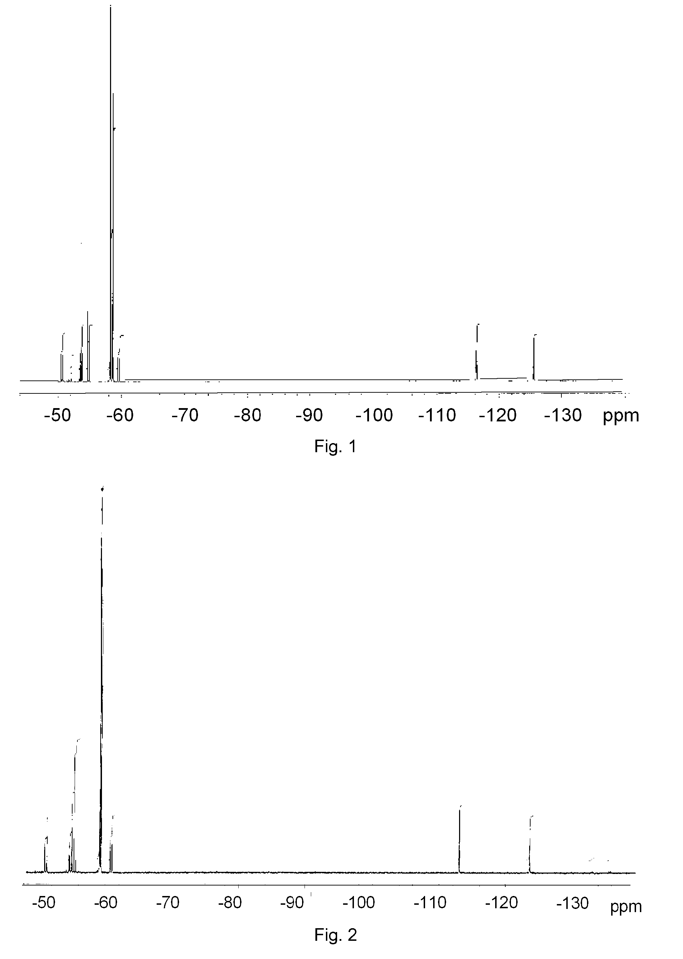

Example 1a. Reaction between perfluoro-5-methoxy-1,3-dioxole (MDO,
(A) in scheme here below) and methanol 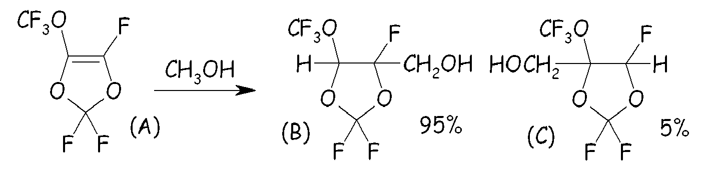 CaCO₃ (7.14 mmol, 0.714 g) was introduced in a stainless steel high pressure vessel equipped with a digital manometer and a magnetic stirrer. After careful evacuation at room temperature, a mixture consisting of CH₃ OH (3.57 moles, 114 g), di-*tert*-butyl peroxide (DTBP; 71.4 mmol, 10.5 g) and MDO (0.714 mol, 150 g) was introduced into the vessel. The vessel was then heated at 134°C under vigorous stirring for 21 hours, by monitoring internal pressure. Once the reaction completed, the vessel was cooled to room temperature and the crude reaction mixture was recovered and rinsed several times with distilled water. The organic (lower) phase is first dried over MgSO₄, filtered and finally distilled. Isolated yield = 56% with respect to the starting MDO (A). b.p. = 142°C. Selectivity = 95% towards target isomer (B); 5% towards alternative isomer (C). Figure 1 depicts the ¹⁹F-NMR spectrum recorded on compound (B). Example 1b. Oxydation of alcohol intermediate (B) 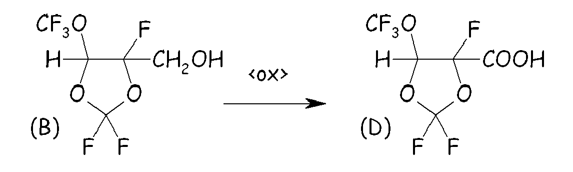 An aqueous solution composed of KMnO₄(238 mmol, 37.6 g), NaOH (238 mmol, 9.52 g) in 200 ml of distilled H₂O was introduced in a 3-necked glass round-bottomed flask equipped with a magnetic stirrer, a dropping funnel, a thermometer and a tap water refrigerating column. The flask was heated to 80°C with vigorous stirring and then 238 mmol; 50 g of product obtained from step 1a was slowly dropped into the basic oxidizing solution. Immediate exothermic release (+ 15°C) was observed together with formation of MnO₂ precipitate. After completion of the addition, solution was further stirred at 80°C for 40 min. Crude reaction mixture was then cooled to room temperature, filtered, acidified to pH = 1 with concentrated HCl (37% w/w) and extracted several times with CH₂Cl₂. The organic layer was dried over MgSO₄, filtered and then the CH₂Cl₂ is evaporated. Isolated yield = 55%, conversion of product from 1 a = 100%. pKₐ of (D) = 2.8.
   Example 1c. Synthesis of VIIa by basic hydrolysis of acid (D) 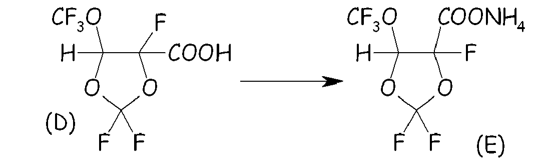 An organic solution composed of (D) (127 mmol; 30.6 g) and 200 ml of CH ₂Cl₂ was introduced in a 2-necked glass round-bottomed flask equipped with a magnetic stirrer, a tap water refrigerating column and a bubbling tube. The mixture was cooled to 0°C with vigorous stirring and a large excess of gaseous NH₃ was bubbled through the organic mixture. Bubbling of NH₃ (g) was pursued until completion of the precipitation of the ammonium salt. Crude mixture was filtered; solid was dried in a vacuum oven at 40°C under reduced pressure (20 mmHg) for 2 hours. A flaky white solid is obtained. Isolated yield of compound (E) (VIIa, with Xₐ being NH₄) = 100%. The thermogravimetric analysis (TGA) in air points out a weight decrease of 10% at 148°C and of 50% at 182°C. Figure 2 depicts the ¹⁹F-NMR spectrum of compound (E). LC-MS analysis showed a strong peak at m/z = 255 (corresponding to the carboxylate mieuty of (E)).

### Preparative Example 2

**Synthesis of compound Va (with Xₐ = NH₄)**

Example 2b Esterification of alcohol intermediate (B) 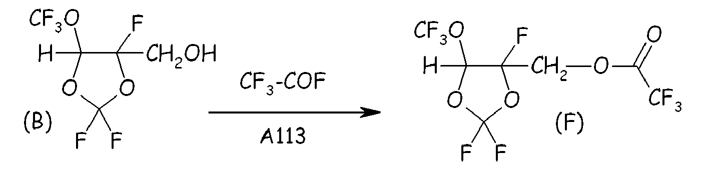 Alcohol intermediate (B) (333 mmol, 70 g) obtained from example 1a was made to react with CF₃COF (350 mmol, 40,5g) in 200 ml of A113 at T = 0°C. Solvent and unreacted CF₃COF were removed by distillation at 40°C/600 mm Hg.

Example 2c Fluorination of ester (F) 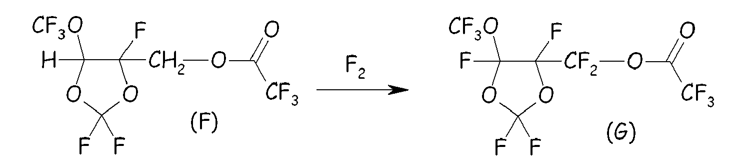 Ester (F) was diluted in A113 (200 ml) and fluorinated with a mixture F₂/N₂ (20/80) at a temperature of 0 to 10°C. Reaction was monitored by gas chromatography. Once fluorination completed, residual F₂ was vented by bubbling a flow of nitrogen. Perfluoroester (G) was recovered after removal under reduced pressure of solvent.

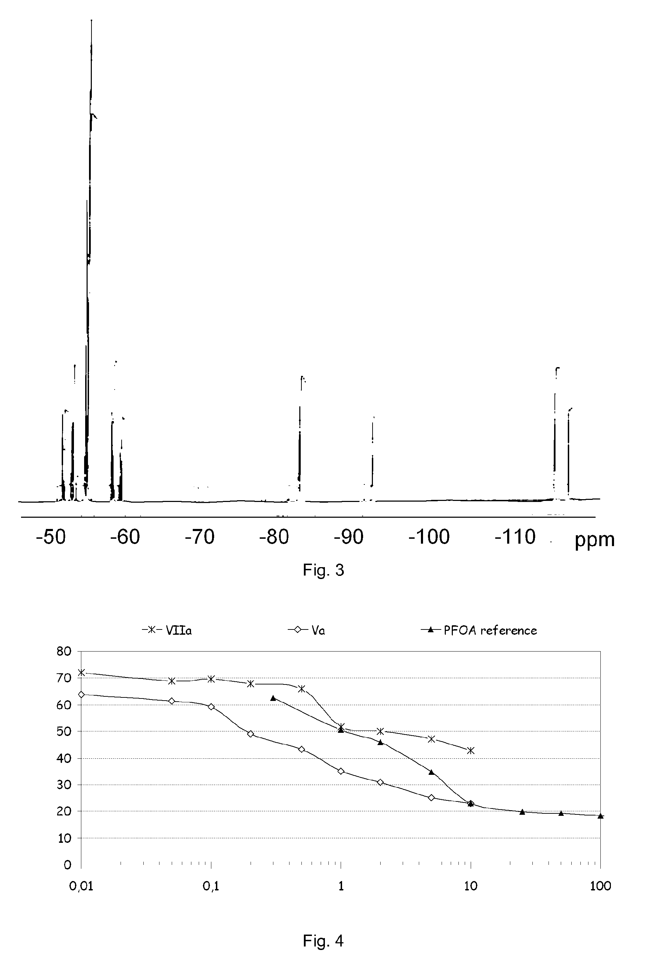

Example 2d Hydrolysis of perfluorinated ester (G) 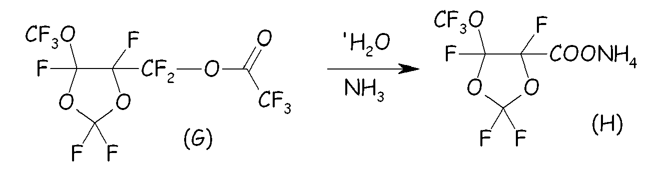 Perfluorinated ester (G) was hydrolyzed in water at 0°C, yielding corresponding acid with quantitative yield. Evolved HF was neutralized with 1.5 mol eq. of KF, yielding solid KHF₂, which was separated by filtration. After removal of solvent, the free acid (b.p. =160°C) and CF₃ COOH (b.p.=72°C) were separated by fractional distillation. Gaseous NH₃ was then bubbled in a CH₂Cl₂ (200 ml) solution of the acid; ammonium salt (H) (formula Va, with Xₐ = NH₄) was then recovered with a yield of 75 % moles (with respect to alcohol intermediate (B)). The thermogravimetric analysis (TGA) in Air points out a weight decrease of 10% at 145°C and of 50% at 182°C. Figure 3 depicts the ¹⁹F-NMR spectrum recorded on compound (H).

**Determination of surface tension of aqueous solution of compounds Va and VIIa (with Xₐ = NH₄)**

Surface tension measurements have been carried out on diluted solutions of ammonium salts of compounds (Va) and (VIIa) in water at a temperature of 25°C, using a LAUDA TE1C tensiometer equipped with a Pt ring; raw data have been worked up with Huh-Mason technique. For comparison purposes, surface tension has been also determined in same conditions on water solution of ammonium perfluorooctanoate (APFO). A sketch of the surface tension (in mN/m) as a function of concentration (in g/I) for compounds Va, VIIa and APFO is given in Figure 4.

**Simulation using density functional theory for prediction of biopersistence behaviour of compound Va**

Using density functional theory, minimum energy conformational structure of carboxylated anion of compound (Va) either in vacuum or in aqueous solution have been determined; in particular, volume and surface of the molecule in solution, solvatation free energy, length of main chain of the molecule, vibrational entropy, equivalent diameter, electrical charges at the oxygen and carbon atoms of the carboxylic groups, dipole momentum have been determined.
Structural and energetic data have been also calculated as above described for several fluorosurfactants such as perfluorooctanoate and other compounds having catenary oxygen atoms, for which biopersistence data were available, So as to establish appropriate correlations among said data and biopersistence profile. In particular, the ratio between solvatation free energy and the length of the molecule has been found to directly correlate to the fraction (%) of compound eliminated from a living animal in rats after 96 hours from administration, as determined by urine analysis.
On the basis of said relation, it has been possible to determine a recovery/elimination for cyclic compound of formula (Va) exceeding 95 % after 96 hours from administration, while only 5 % are expected to be rejected from living body from similar calculations from perfluorooctanoate. These data well demonstrate that the cyclic compounds of the invention indeed possess a more favourable biopersistence profile over traditional fluorosurfactants.

**Blood levels and pharmacokinetic parameters of compound VIIa (Xₐ = NH₄ )**

Compound VIIa (Xₐ = NH₄) was administered by single oral route (gavage) to three male Wistar (SPF-bred) rats at dose levels of 73 µmol/kg of dry ammonium salt, corresponding to 19.9 mg/kg. Blood sampling occurred 15 minutes before administration, at 4, 8, 12, 24, 72 and 168 hours after administration. Maximal plasma concentrations (Cₘₐₓ₎ was observed at 4 h (tₘₐₓ) and the half life of 17 hours was calculated after oral administration. The PK parameters of this compound after single oral (gavage) administration to male rats are summarized in the table below:

**Table 1**

| | | |
|---|---|---|
| Dose [mg/kg] | | 19.9 |
| Males | | Group (plasma) |
| Cₘₐₓ | [ng/mL] | 14977 |
| tₘₐₓ | [h] | 4 |
| AUC₀₋ₜ | ] [ng•h/mL ] | 101677 |
| AUC_{inf.} | [ng•h/mL ] | [101915] |
| t_{½, z} | [h] | [17] |

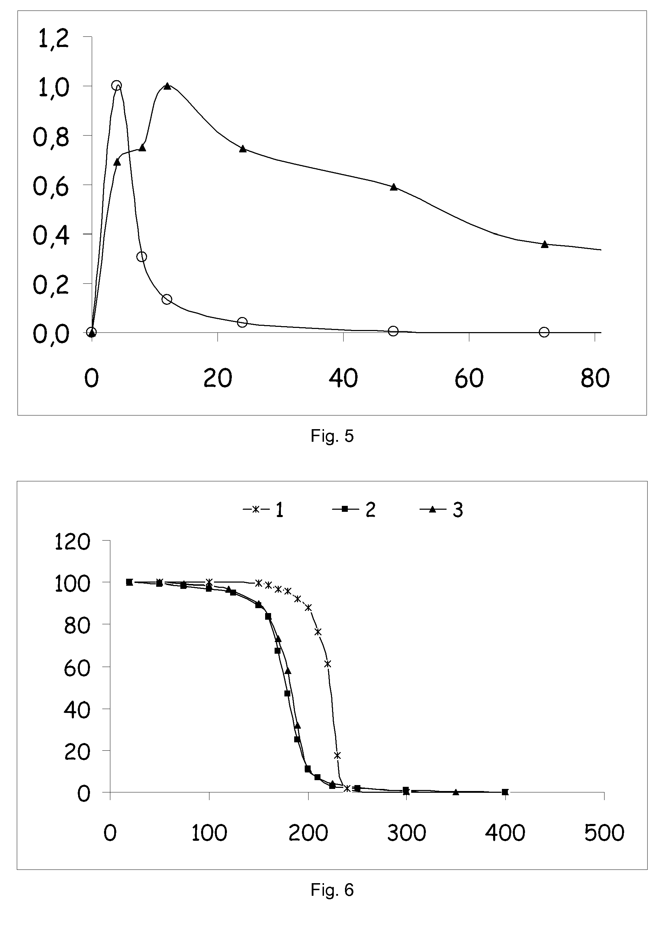

Results of plasma concentrations for compound VIIa (Xₐ = NH₄) and APFO as a function of time are sketched in Figure 5. This graph depicts ratio C/C ₘₐₓ for compound VIIa (Xₐ = NH₄) (O) and APFO(▲ ), with C= instantaneous plasma concentration and Cₘₐₓ = maximum plasma concentration, as a function of time (in hours). Experimental data indicate significantly faster elimination of compound VIIa (Xₐ = NH₄) from blood after single oral administration than what observed for APFO.

**TGA analyses of Compounds Va and VIIa (Xₐ = NH₄) and APFO as comparison**

Figure 6 depicts the TGA traces as % wt loss as a function of temperature (in °C) for APFO (1), compound Va (2) and VIIa (Xₐ = NH₄) (3). These data well demonstrate that cyclic compounds are more volatile that perfluoroalkanoic acids and thus are expected to leave lower levels of residues in final parts obtained from dispersions containing the same.

**Polymerization Example 3: PTFE polymerization in the presence of compound Va (Xₐ = NH₄)**

A polymerization reactor having a total volume of 100 cc equipped with a mechanical stirrer was charged with 60 cc of deionised water, 0.12 g of compound Va (Xₐ = NH₄) and 1.0 g of paraffin wax with softening point comprised 52°C and 58°C. The reactor was evacuated and heated up to 70°C. The reactor was kept under mechanical stirring and loaded with gaseous TFE until reaching a pressure of 20 barg. The polymerization was initiated by a solution containing 0.5 mg of ammonium peroxodisulfate (NH ₄)₂S₂O₈ (APS) and 9.6 mg of disuccinic acid peroxide (DSAP). Reaction pressure was maintained at set point of 20 barg by feeding gaseous TFE. The reaction temperature was increased until 80°C with a rate of 0.5°C/min. After 80 min, feeding of TFE was interrupted, reactor was vented and cooled. A stable PTFE dispersion having a solid content of 20% wt was obtained; no coagulum was formed in the reactor during polymerization. The latex particle diameter was found to be 235 nm when measured by Laser Light Scattering (LLS).

## Claims

1. A method for making a fluoropolymer comprising an aqueous emulsion polymerization of one or more fluorinated monomers wherein said aqueous emulsion polymerization is carried out in the presence of at least one cyclic fluorocompound of the following formula (I): 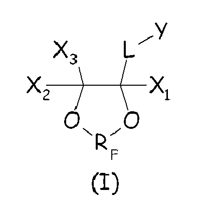 wherein X₁, X₂, X₃, equal or different from each other are independently selected among H, F, and C₁₋₆ (per)fluoroalkyl groups, optionally comprising one or more catenary or non-catenary oxygen atoms; L represents a bond or a divalent group; R_{F} is a divalent fluorinated C₁₋₃ bridging group; Y is a hydrophilic function selected among anionic functionalities, cationic functionalities and non-ionic functionalities.

2. The method of claim 1, wherein the hydrophilic function Y of cyclic fluorocompound (I) is selected among non-ionic functions of formulae -(OR_{H})ₙ -OH, wherein R_{H} is a divalent hydrocarbon group, and n is an integer of 1 to 15.

3. The method of claim 1, wherein the hydrophilic function Y of cyclic fluorocompound (I) is selected among cationic functions of formulae: 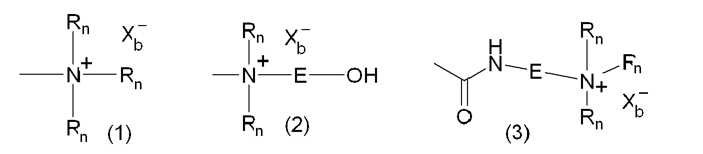 wherein Rₙ, equal or different at each occurrence, represents an hydrogen atom or a C₁₋₆ hydrocarbon group (preferably an alkyl group), E is a C₁₋₃ divalent hydrocarbon group and X_{b}⁻ is an anion selected among OH⁻, Cl⁻, Br-, I⁻

4. The method of claim 1, wherein the hydrophilic function Y of cyclic fluorocompound (I) is selected among anionic functions, in particular among those of formulae: 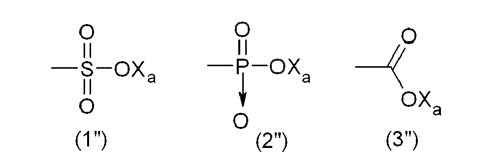 wherein Xₐ is H, a monovalent metal (preferably an alkaline metal) or an ammonium group of formula -N(R'ₙ)₄, wherein R'ₙ, equal or different at each occurrence, represents a hydrogen atom or a C₁₋₆ hydrocarbon group.

5. The method of claim 4, wherein the cyclic fluorocompound complies with formula (II) here below: 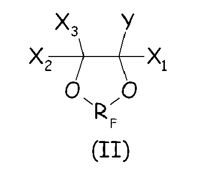 wherein X₁, X₂, X₃, Y and R_{F} have the same meaning as in Claim 1.

6. The method of claim 5, wherein the cyclic fluorocompound complies with formula (IV): 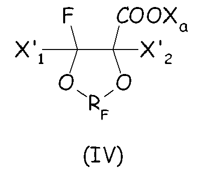 wherein X'₁ and X'₂ , equal or different from each other, are independently a fluorine atom, a -R'_{f} group or -OR'_{f} group, wherein R'_{f} is a C₁₋₃ perfluoroalkyl group, and R_{F} and Xₐ have the same meanings as in Claim 1 and 4, respectively.

7. The method of claim 5, wherein the cyclic fluorocompound complies with formula (VI) here below: 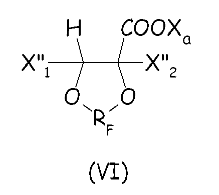 wherein X"₁ and X"₂, equal or different from each other, are independently a fluorine atom, a -R'_{f} group or -OR'_{f} group, wherein R'_{f} is a C₁₋₃ perfluoroalkyl group, and R_{F} and Xₐ have the same meanings as in Claim 6.

8. The method of claim 4, wherein the cyclic fluorocompound complies with formula (VIII) here below: 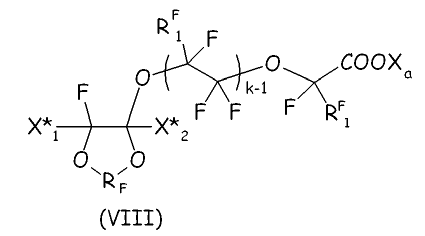 wherein R^{F} and Xₐ have the same meanings as in Claim 1 and 4, respectively; X*₁, X*₂ equal or different each other are independently a fluorine atom, -R'_{f} or -OR'_{f}, wherein R'_{f} is a C₁₋₃ perfluoroalkyl group; R^{F}₁ is F or CF₃, k is an integer from 1 to 3.

9. Cyclic fluorocompounds of formula (I) 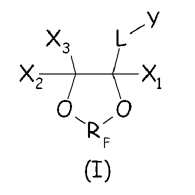 wherein X₁, X₂, X₃, equal or different from each other are independently selected among H, F, and C₁₋₆ (per)fluoroalkyl groups, optionally comprising one or more catenary or non-catenary oxygen atoms; L represents a bond or a divalent group; R_{F} is a divalent fluorinated C₁₋₃ bridging group; Y is a hydrophilic function selected among anionic functionalities, cationic functionalities and non-ionic functionalities.

10. Cyclic fluorocompounds of claim 9, complying with formula (IV) or (VI): 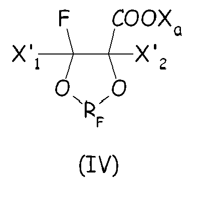 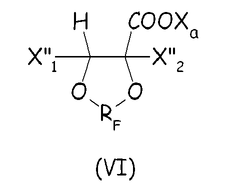 wherein X'₁ , X'_{2 ,} X"₁ and X"_{2,} equal or different from each other, are independently a fluorine atom, a -R'_{f} group or -OR'_{f} group, wherein R'_{f} is a C₁₋₃ perfluoroalkyl group, and R_{F} has the same meanings as in Claim 9, Xₐ is H, a monovalent metal (preferably an alkaline metal) or an ammonium group of formula -N(R'ₙ)₄, wherein R'ₙ, equal or different at each occurrence, represents a hydrogen atom or a C₁₋₆ hydrocarbon group.

11. Cyclic fluorocompounds of claim 9, complying with formula (VIII) here below: 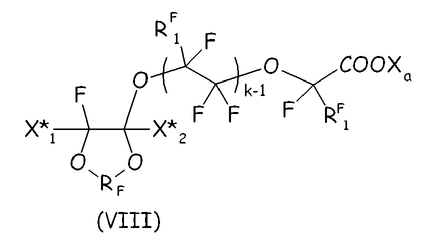 wherein R^{F} and Xₐ have the same meanings as in Claim 10; X*₁, X*₂ equal or different each other are independently a fluorine atom, -R'_{f} or -OR'_{f,} wherein R' _{f} is a C₁₋₃ perfluoroalkyl group; R^{F}₁ is F or CF₃, k is an integer from 1 to 3.

12. Fluoropolymer dispersions comprising at least one cyclic fluorocompound of the formula (I) 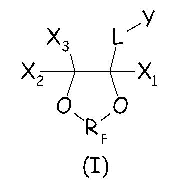 wherein X₁, X₂, X₃, equal or different from each other are independently selected among H, F, and C₁₋₆ (per)fluoroalkyl groups, optionally comprising one or more catenary or non-catenary oxygen atoms; L represents a bond or a divalent group; R_{F} is a divalent fluorinated C₁₋₃ bridging group; Y is a hydrophilic function selected among anionic functionalities, cationic functionalities and non-ionic functionalities.
